# FASCICULE DE BREVET EUROPEEN

(11) **EP 1 266 947 B1**
(45) Date de publication et mention de la délivrance du brevet: **04.08.2004**
(21) Numéro de dépôt: 01490022.9
(22) Date de dépôt: 15.06.2001
(51) Int. Cl.: C09J 5/06, A61F 5/14, A61F 5/01

(54) **Procédé de fabrication d'une pièce orthopédique à base d'élastomère de silicone et pièce orthopédique obtenue**
Verfahren zur Herstellung eines orthopädischen Gegenstandes auf Basis eines Silikonelastomers und so hergestellter orthopädischer Gegenstand
Method for producing a silicon elastomer based orthopedic object and orthopedic object produced thereby

(43) Date de publication de la demande: 18.12.2002
(73) Titulaire: THUASNE, 92300 Levallois-Perret (FR)
(72) Inventeur: Sterna, Laurent, 69007 Lyon (FR)
(74) Mandataire: Hennion, Jean-Claude

(56) Documents cités:
- EP-A- 0 495 152

## Description

La présente invention concerne le domaine des matériels orthopédiques que ce soient des semelles ou des talonnettes ou des orthèses, ces matériels ayant en commun de mettre en oeuvre un matériau textile et un élément compressible. Elle concerne plus particulièrement un procédé de fabrication d'une pièce orthopédique qui comprend un bloc en élastomère de silicone et un matériau textile protecteur. Elle concerne également la pièce orthopédique obtenue par le procédé.

Dans le domaine des orthèses pour articulations, on connaît par exemple par le document EP.0.360.277 un bandage ou manchon réalisé dans une matière textile élastique qui comporte un élément de rembourrage, c'est-à-dire un élément susceptible d'améliorer le maintien en place de l'articulation, ledit élément étant rendu solidaire du bandage proprement dit grâce à un revêtement textile qui le recouvre. L'élément de rembourrage peut en particulier être réalisé dans un matériau élastomère, éventuellement en silicone. L'élément de rembourrage est emprisonné entre le bandage proprement dit qui est en contact avec la peau de l'utilisateur et le revêtement qui le recouvre. La fixation de ce revêtement sur le bandage se fait soit par soudage soit par couture des bords du revêtement dépassant au-delà du rembourrage. Dans ce type d'orthèse, le rembourrage n'est pas directement fixé sur le bandage.

Dans certains types d'orthèses telles que des semelles ou talonnettes, il peut être intéressant que le matériau textile protecteur soit fixé directement sur l'élément compressible. Pour que cette fixation soit continue sur l'ensemble de la surface du matériau textile , il est bien naturel d'envisager une fixation par collage. Cependant on sait que l'élastomère de silicone est un matériau qui est difficile à coller. Les colles habituelles du type cyanoacrylate ne sont guère satisfaisantes dans la mesure où elles sont cassantes, non-élastiques et particulièrement coûteuses. Le fait que ces colles du type cyanoacrylate n'aient pas de propriétés élastiques est un inconvénient majeur dans le domaine des orthèses puisque le plus généralement le matériau textile utilisé est un matériau élastique qui doit pouvoir suivre les déformations de l'élément compressible lors des différents mouvements de l'utilisateur.

Le but que s'est fixé le demandeur est de proposer un procédé de fabrication d'une pièce orthopédique qui comprend un bloc d'une matière compressible et un matériau textile protecteur qui pallient les inconvénients précités.

De manière caractéristique , le bloc étant en élastomère de silicone, le procédé consiste :
a) à mélanger les deux composants d'une colle de silicone bi-composant en sorte d'obtenir une pâte de haute viscosité,
b) à enduire une face du matériau textile à l'aide d'une quantité réduite de ladite pâte,
c) à appliquer la face enduite du matériau textile sur le bloc en élastomère de silicone et
d) à chauffer l'ensemble à une température et pendant un temps suffisants pour obtenir la réticulation de la colle de silicone.

Pour la réalisation de matériels orthopédiques tels que des semelles ou talonnettes, le bloc en élastomère de silicone est obtenu par moulage. De préférence , dans ce cas, l'application de la face enduite du matériau textile sur le bloc moulé est réalisée à bref délai après le démoulage dudit bloc. En effet, il est possible qu'il reste encore des sites libres de réticulation sur le bloc de silicone fraîchement démoulé et que ces sites puissent interagir avec les composants de la colle de silicone lors de la réticulation de celle-ci, ce qui tend à améliorer encore les propriétés d'adhésion entre la colle de silicone et le bloc d'élastomère en silicone.

L'enduction du matériau textile a de préférence la configuration de la surface du bloc en élastomère de silicone sur laquelle ledit matériau textile enduit est appliqué. Dans ce cas on réalise l'enduction à l'aide d'un pochoir ayant ladite configuration, notamment un cadre sérigraphique à mailles ouvertes.

Cependant il est possible, le matériau textile se présentant sous la forme d'une bande de grande longueur, de réaliser l'enduction en continu de manière uniforme sur toute la largeur de ladite bande ; dans ce cas , on réalise dans ladite bande enduite une découpe à la forme de la face du bloc compact. Cette découpe est réalisée soit avant soit après l' opération de chauffage. Dans ce dernier cas, elle est facilitée du fait que l'outil de coupe n 'est pas souillé par la colle à l'état pâteux.

La présente invention sera mieux comprise à la lecture de la description qui va être faite de deux exemples de réalisation d'un matériel orthopédique comportant un bloc compact en élastomère de silicone solidaire d'un matériau textile protecteur, à savoir d'une part une semelle orthopédique et d'autre part une orthèse pour l'articulation du genou , illustré par le dessin annexé dans lequel :
La figure 1 est une vue en plan de dessus de la semelle orthopédique,
La figure 2 est une vue en plan de dessous de la semelle orthopédique,
La figure 3 est une vue en coupe selon l'axe III-III de la figure 1,
Les figures 4 à 6 illustrent partiellement les étapes du procédé de fabrication de la semelle orthopédique,
La figure 7 est une vue partielle en plan d'une orthèse pour l'articulation du genou.

Le principe de l'invention est de réaliser un matériel orthopédique comprenant un élément compressible et un matériau textile en mettant en oeuvre comme élément compressible un élastomère de silicone et en solidarisant ledit élément compressible avec le matériau textile à l'aide d'une colle de silicone qui est également un élastomère de silicone éventuellement d'une nature différente.

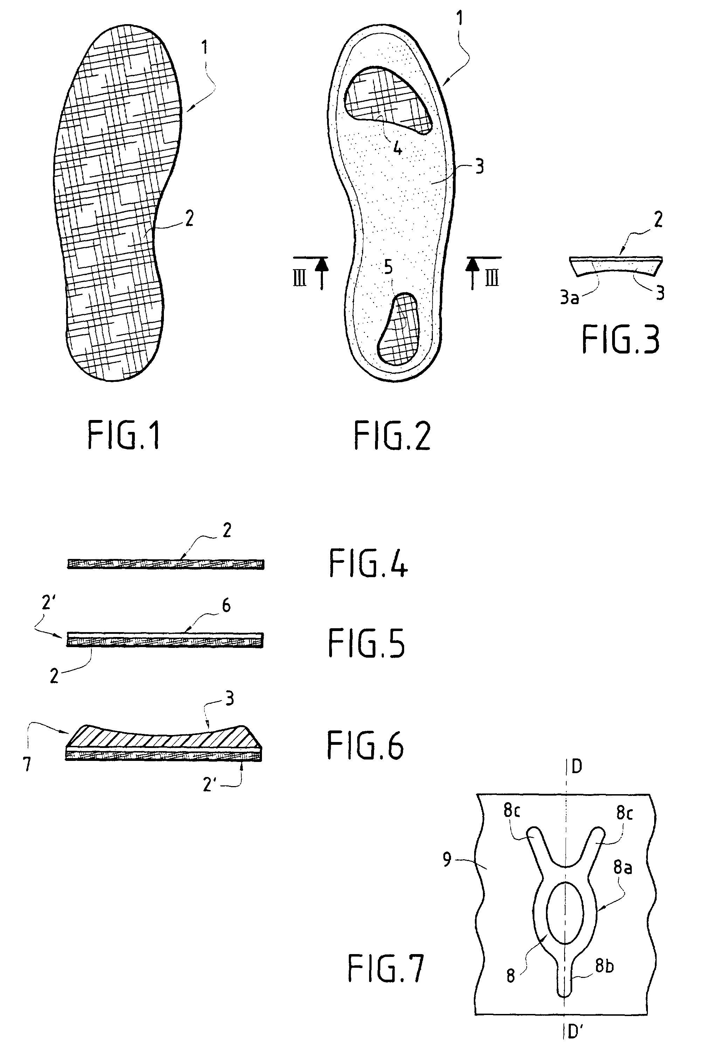

Selon le premier exemple, illustré aux figures 1 à 6, le matériel orthopédique est une semelle 1 orthopédique sur laquelle est solidarisé un non-tissé 2 en polyamide non élastique faisant office de revêtement protecteur, c'est-à-dire de revêtement qui est destiné à être en contact avec le pied de l'utilisateur.

Cet exemple n'est pas exclusif de l'invention. Le matériel orthopédique peut consister en une talonnette ou également en une orthèse du type genouillère, chevillère , coudière...D'ailleurs le second exemple qui sera décrit ci-après concerne une genouillère, c'est-à-dire une orthèse pour l'articulation du genou. De plus le revêtement protecteur peut être non seulement un non-tissé mais également un tissu ou un tricot , plus particulièrement lorsqu'il s'agit d'un revêtement dont on souhaite des propriétés élastiques.

Dans la semelle orthopédique 1 , le revêtement protecteur en non-tissé 2 est solidarisé au bloc 3 en élastomère de silicone grâce à une colle silicone 6 à deux composants.

Le bloc 3 en élastomère de silicone est réalisé par moulage à partir d'un silicone de type RTV (de l'anglais Room Temperature Vulcanization) plus particulièrement RTV 2 c'est-à-dire à deux composants, obtenu par polyaddition avec une catalyse par le platine. Ce silicone est particulièrement souple , ayant une dureté shore A inférieure ou égale à 50. Le bloc 3 en élastomère de silicone est mis en forme dans un moule ayant la configuration souhaitée pour la semelle, telle que cette configuration ressort à l'examen des figures 2 et 3. Ce bloc 3 a une face sensiblement plane 3a, qui est celle sur laquelle doit être solidarisé le non-tissé 2 . Par contre son épaisseur n'est pas uniforme et son flanc latéral périphérique 3b présente une inclinaison variable. Toutes ces conditions de réalisation sont fonction du type de semelle. Egalement, comme cela est illustré à la figure 2 , peuvent être prévus des évidements 4, 5 , dans les zones d'appui de la semelle 1 , lesquels évidements étant ultérieurement remplis d'un autre matériau compressif , éventuellement également en élastomère de silicone de propriétés différentes.

La solidarisation du non-tissé 2 sur le bloc 3 en élastomère de silicone est réalisée grâce à une colle de silicone 6, du type LSR qui est une famille de silicone bi-composant très visqueux. Dans un exemple précis de réalisation, il s'agissait d'une colle se réticulant par polyaddition à catalyse de platine. Pour cela on prépare les deux composants de ladite colle en les mélangeant pour obtenir une pâte de haute viscosité, par exemple de l'ordre de 700.000mPa.s. La haute viscosité de la colle permet de réaliser sur le non-tissé 2 une enduction sans qu'il y ait pénétration de celle-ci à travers ledit non-tissé. Cet effet est obtenu pour une viscosité minimale de 300.000mPa.s. Pour réaliser cette enduction , on met en oeuvre , en sortie de la machine conventionnelle réalisant le dosage et le mélange des deux composants une buse dont l'écartement des lèvres est de l' ordre de ou inférieur à 1mm, ladite buse ayant une longueur fonction de la largeur souhaitée pour l'enduction. Le mélange visqueux sort de la buse sous forme d'une nappe très fine. S'agissant d'une enduction continue sur une bande du non-tissé , il suffit de déplacer ladite bande à proximité immédiate de la buse pour que ladite nappe s'étale régulièrement sur le non-tissé. On peut régler facilement la quantité de colle ainsi déposée sur le non-tissé en réglant d'une part les conditions opératoires du dispositif de dosage et de mélange et d'autre part les conditions opératoires de déplacement de la bande par rapport à la buse , tant en distance par rapport à la buse qu'en vitesse. Le non-tissé 2' enduit avec la colle 6 est appliqué sur la face sensiblement plane 3a du bloc 3 en élastomère de silicone.

L'ensemble ainsi constitué par le non-tissé 2, la colle de silicone 6 et le bloc en élastomère de silicone 3 est placé dans un four en sorte d'obtenir la réticulation de la colle de silicone 6. Il peut notamment s'agir d'un chauffage progressif allant de 80°C jusqu'à 200°C. Bien sûr les conditions de cette réticulation et en particulier la température et le temps de chauffage sont déterminées en fonction du matériau dont est constitué le non-tissé ou plus généralement le matériau textile faisant office de revêtement protecteur. On comprend que la température de réticulation doit être choisie pour qu'il n'y ait pas de dégradation dudit matériau textile. Le non-tissé 2' est ensuite découpé suivant le contour du bloc 3.

Dans un exemple précis de réalisation, le non-tissé était en polyamide. L'adhérence obtenue entre le non-tissé 2 et le bloc 3 en élastomère de silicone était bonne. Les essais dynamométriques ont montré une rupture cohésive.

Comme indiqué précédemment le bloc en élastomère de silicone 3 est obtenu par moulage. En pratique, il est préférable que l'étape d'application de la face enduite du matériau textile sur ledit bloc intervienne dans un temps court après le démoulage du bloc. On a en effet constaté que l'adhérence obtenue dans ces conditions était relativement meilleure que si l'application de la face enduite du matériau textile sur le bloc en élastomère de silicone se faisait avec un bloc réticulé et démoulé depuis longtemps. Le demandeur peut tenter d'expliquer ce phénomène en supposant qu'il reste sur le bloc fraîchement démoulé des sites libres de réticulation qui peuvent réagir avec les sites portés par les composants de la colle de silicone.

En tout état de cause, il est préférable de réaliser l'application à bref délai, en particulier pour les blocs en élastomère de silicone relativement mous , notamment ayant une dureté shore A égale ou inférieure à 15. En effet le demandeur a constaté que dans le temps de l'huile de silicone pouvait exsuder de la surface de tels blocs et gêner le collage. Dans ce cas, le bref délai ne doit pas dépasser 1 heure.

Dans le second exemple de réalisation qui est illustré à la figure 7, il s 'agit d'une orthèse pour l'articulation du genou dans laquelle le bloc 8 en élastomère de silicone est collé sur un manchon 9 réalisé dans un tissu ou un tricot élastique dans les deux sens, en viscose-polyester. Il est bien sûr nécessaire que la colle en silicone qui est utilisée pour réaliser la solidarisation du bloc 8 sur le manchon 9 soit elle-même déformable élastiquement, dans des conditions qui soient comparables à la déformation obtenue pour le bloc en élastomère de silicone 8. On choisit en conséquence le grade de la colle du silicone et la quantité mise en oeuvre.

Dans le mode de réalisation qui est illustré sur la figure 7 , le bloc 8 en élastomère de silicone a un axe longitudinal de symétrie DD'. Il comporte une partie centrale 8a de forme annulaire et sensiblement ovale ainsi que trois prolongements , l'un 8b selon l'axe DD' et les deux autres 8c disposés à l'opposé du premier prolongement 8b et écartés angulairement l'un de l'autre , symétriquement par rapport à l'axe DD'.

Au contraire de ce qui a été réalisé pour la semelle 1 du premier exemple, le matériau textile a une configuration extérieure qui diffère de celle du bloc compact. Ceci nécessite de pouvoir réaliser l 'enduction de colle 6 de silicone sur le tissu ou le tricot élastique 9 selon la configuration exacte du bloc 8. Ceci est obtenu en utilisant le même matériel que précédemment, avec la buse d'enduction mais en mettant en oeuvre un pochoir que l'on place entre la buse et le tissu ou le tricot. La colle est ainsi déposée suivant le dessin du pochoir, dessin qui correspond à la configuration de la face du bloc 8 qui doit être collé sur le tissu ou le tricot. Ce pochoir peut notamment être un cadre de sérigraphie à mailles très ouvertes.

Il est à noter que l'application de la face enduite du matériau textile sur le bloc en élastomère de silicone peut être réalisée alors que le bloc n'est pas ou pas totalement réticulé , étant encore placé dans son moule. S'agissant en particulier de la fabrication de la semelle selon le premier exemple, le matériau textile prédécoupé et enduit de la colle de silicone, peut être placé dans le fond du moule avant injection de l'élastomère de silicone. Lors du moulage le moule est porté à une température permettant la réticulation de l'élastomère en silicone et on obtient, simultanément, la réticulation également de la colle de silicone.

Il est à souligner que grâce à la présence de la colle de silicone, préalablement enduite sur le matériau textile , on n'obtient pas de transpercement dudit matériau textile, ce qui aurait été un risque majeur dans le cas où l'on aurait procédé directement au moulage de l'élastomère de silicone sur le matériau textile, la colle de silicone formant une barrière empêchant la pénétration de l'élastomère en silicone entre les fils ou fibres constitutifs du matériau textile.

Le bloc en élastomère de silicone peut éventuellement être d'un silicone de la même famille que celui de la colle de silicone et être obtenu par moulage à chaud sur presse à injecter.

A titre d'exemple non limitatif , la composition de la colle de silicone de type LSR et du silicone du type RTV 2 comprend un premier composant à base de polydiméthylsiloxane + polydiméthylvinylsiloxane + charges + sels de platine et un second composant à base de polydiméthylsiloxane + polydiméthylhydrogénosiloxane + charges.

## Revendications

1. Procédé de fabrication d'une pièce orthopédique comprenant un bloc d'une matière compressible et un matériau textile protecteur, **caractérisé en ce que** , le bloc étant en élastomère de silicone, il consiste :
a) à mélanger les deux composants d'une colle de silicone bi-composant en sorte d'obtenir une pâte de haute viscosité,
b) à enduire une face du matériau textile à l'aide d'une quantité réduite de ladite pâte,
c) à appliquer la face enduite du matériau textile sur le bloc en élastomère de silicone et
d) à chauffer l'ensemble à une température et pendant un temps suffisants pour obtenir la réticulation de la colle de silicone.

2. Procédé selon la revendication 1 **caractérisé en ce que** la colle de silicone est une pâte dont la viscosité est d'au moins 300.000mPa.s

3. Procédé selon la revendication 2 **caractérisé en ce qu'**il s'agit d'une colle de silicone de type LSR ayant une viscosité de l'ordre de 700.000mPa.s.

4. Procédé selon l'une des revendications 1 à 3 **caractérisé en ce que** le bloc en élastomère de silicone est un silicone de type RTV 2.

5. Procédé selon l'une des revendications 1 à 4 **caractérisé en ce que** le bloc en élastomère de silicone a une dureté shore A inférieure ou égale à 50.

6. Procédé selon l'une des revendications 1 à 5 **caractérisé en ce que**, le bloc en élastomère de silicone étant obtenu par moulage, l'application de la face enduite du matériau textile sur le bloc moulé est réalisée à bref délai après le démoulage dudit bloc.

7. Procédé selon la revendication 6 **caractérisé en ce que** le bloc moulé en élastomère de silicone a une dureté shore A inférieure ou égale à 15.

8. Procédé selon la revendication 7 **caractérisé en ce que** le bref délai est inférieur à une heure.

9. Procédé selon l'une des revendications 1 à 8 **caractérisé en ce qu'**on réalise l'enduction en continu sur le matériau textile se présentant sous la forme d'une bande de grande longueur et **en ce qu'**on découpe le matériau textile enduit à la forme de la face du bloc en élastomère de silicone au moins, de préférence après l'opération de chauffage.

10. Procédé selon l'une des revendications 6 à 9 **caractérisé en ce que** l'application de la face enduite du matériau textile sur le bloc en élastomère de silicone est réalisée alors que ledit bloc n'est pas ou pas totalement réticulé , étant encore placé dans son moule.

11. Procédé selon l'une des revendications 9 ou 10 **caractérisé en ce que** l'enduction se fait selon la configuration de la face du bloc d'élastomère en silicone, à l'aide d'un pochoir, notamment un cadre sérigraphique à mailles très ouvertes.

12. Matériel orthopédique obtenu notamment par le procédé selon l'une des revendications 1 à 11, **caractérisé en ce qu'**il comporte un élastomère de silicone collé sur un matériau textile protecteur à l'aide d'une colle de silicone.

13. Matériel orthopédique selon la revendication 12 consistant en une semelle orthopédique **caractérisé en ce que** le matériau textile protecteur est un non-tissé polyamide non-élastique et l'élastomère de silicone est du type RTV 2 ayant une dureté shore A inférieure à 50.

14. Matériel orthopédique selon la revendication 12 consistant en une orthèse pour l'articulation du genou **caractérisé en ce que** le matériau textile est un tissu ou un tricot élastique et **en ce que** l'élastomère de silicone est un élastomère de type RTV 2, ayant une dureté shore A inférieure à 50.

## Patentansprüche

1. Verfahren zum Herstellen eines orthopädischen Teils mit einem Block aus einem komprimierbaren Material und mit einem textilen Schutzmaterial, **dadurch gekennzeichnet, dass** es - wobei der Block aus Silikonelastomer besteht - darauf beruht:
a) die beiden Komponenten eines Zweikomponenten-Silikonklebers zu mischen, so dass eine Masse mit hoher Viskosität erhalten wird,
b) eine Seite des textilen Materials mit Hilfe einer begrenzten Menge der genannten Masse zu beschichten
c) die beschichtete Seite des textilen Materials auf den Block aus Silikonelastomer aufzulegen und
d) das Ganze auf eine Temperatur und für eine Zeit zu erhitzen, die hoch bzw. lang genug ist, um die Vernetzung des Silikonklebers zu erzielen.

2. Verfahren nach Anspruch 1, **dadurch gekennzeichnet, dass** der Silikonkleber eine Masse ist, deren Viskosität wenigstens 300.000mPa.s beträgt.

3. Verfahren nach Anspruch 2, **dadurch gekennzeichnet, dass** es sich um einen Silikonkleber vom Typ LSR handelt, der eine Viskosität in der Größenordnung von 700.000mPa.s aufweist.

4. Verfahren nach einem der Ansprüche 1 bis 3, **dadurch gekennzeichnet, dass** der Block aus Silikonelastomer ein Silikon vom Typ RTV 2 ist.

5. Verfahren nach einem der Ansprüche 1 bis 4, **dadurch gekennzeichnet, dass** der Block aus Silikonelastomer eine Shorehärte A kleiner oder gleich 50 aufweist.

6. Verfahren nach einem der Ansprüche 1 bis 5, **dadurch gekennzeichnet, dass** - da der Block aus Silikonelastomer durch Formen erhalten wird - das Auflegen der beschichteten Seite des textilen Materials auf den geformten Block kurze Zeit nach dem Ausformen des genannten Blocks vollzogen wird.

7. Verfahren nach Anspruch 6, **dadurch gekennzeichnet, dass** der geformte Block aus Silikonelastomer eine Shorehärte A kleiner oder gleich 15 aufweist.

8. Verfahren nach Anspruch 7, **dadurch gekennzeichnet, dass** die kurze Zeit weniger als eine Stunde beträgt.

9. Verfahren nach einem der Ansprüche 1 bis 8, **dadurch gekennzeichnet, dass** das textile Material, welches in Form eines Streifens großer Länge vorliegt, stetig beschichtet wird und dass das beschichtete textile Material wenigstens, vorzugsweise nach dem Schritt des Erhitzens, auf die Form der Seite des Blocks aus Silikonelastomer zugeschnitten wird.

10. Verfahren nach einem der Ansprüche 6 bis 9, **dadurch gekennzeichnet, dass** das Auflegen der beschichteten Seite des textilen Materials auf den Block aus Silikonelastomer vollzogen wird, noch während der genannte Block nicht oder nicht vollkommen vernetzt ist, wobei er sich noch in seiner Form befindet.

11. Verfahren nach einem der Ansprüche 9 oder 10, **dadurch gekennzeichnet, dass** das Beschichten entsprechend der Gestaltung der Seite des Blocks aus Silikonelastomer mit Hilfe einer Schablone, insbesondere einem Siebdruckrahmen mit weit geöffneten Maschen erfolgt.

12. Orthopädisches Material, das insbesondere durch das Verfahren nach einem der Ansprüche 1 bis 11 erhalten wird, **dadurch gekennzeichnet, dass** es ein Silikonelastomer aufweist, das mit Hilfe eines Silikonklebers auf ein textiles Schutzmaterial aufgeklebt ist.

13. Orthopädisches Material nach Anspruch 12, bestehend aus einer orthopädischen Sohle, **dadurch gekennzeichnet, dass** das textile Schutzmaterial ein unelastisches Polyamidvlies ist und dass das Silikonelastomer vom Typ RTV 2 mit einer Shorehärte A kleiner 50 ist.

14. Orthopädisches Material nach Anspruch 12, bestehend aus einer Orthese für das Kniegelenk, **dadurch gekennzeichnet, dass** das textile Material ein Gewebe oder ein elastischer Trikot ist und dass das Silikonelastomer ein Elastomer vom Typ RTV 2 mit einer Shorehärte A kleiner 50 ist.

## Claims

1. A process for the manufacture of an orthopaedic product consisting of a block made of a compressible material and a protective textile material, wherein, the block being made of a silicone elastomer, it consists in:
a) mixing two components of a two-component silicone adhesive in order to obtain a high-viscosity paste,
b) coating one surface of a textile material with a reduced quantity of the said paste,
c) applying the coated surface of the textile material to the silicone elastomer block, and
d) heating the unit to a sufficient temperature and for a sufficient period of time to enable cross-linking of the silicone adhesive to be achieved.

2. A process according to claim 1, wherein the silicone adhesive is a paste having a viscosity of at least 300,000 mPa s.

3. A process according to claim 2, wherein the silicone adhesive is an LSR-type silicone adhesive having a viscosity of approximately 700,000 mPa s.

4. A process according to any one of claims 1 to 3, wherein the silicone elastomer block is an RTV-2-type silicone.

5. A process according to any one of claims 1 to 4, wherein the silicone elastomer block has a Shore hardness A equal to or less than 50.

6. A process according to any one of claims 1 to 5, wherein, the silicone elastomer block being moulded, the application of the coated surface of the textile material to the moulded block is performed within a short period following removal of said block from the mould.

7. A process according to claim 6, wherein the moulded silicone elastomer block has a Shore hardness A equal to or less than 15.

8. A process according to claim 7, wherein the short period is less than one hour.

9. A process according to any one of claims 1 to 8, wherein the coating of the textile material is performed continuously, said textile material being in the form of a long strip, and wherein the coated textile material is cut out in the shape of the surface of the silicone elastomer block at least, preferably following the heating operation.

10. A process according to any one of claims 6 to 9, wherein the application of the coated surface of the textile material to the silicone elastomer block is performed while the block is not or is not completely cross-linked, being still positioned in its mould.

11. A process according to claim 9 or 10, wherein the coating is performed according to the general shape of the surface of the silicone elastomer block by means of a stencil, particularly a coarse open-mesh screen-printing frame.

12. An orthopaedic device obtained particularly through the process according to any one of claims 1 to 11, wherein it consists of a silicone elastomer glued to a protective textile material by means of a silicone adhesive.

13. An orthopaedic device according to claim 12, consisting of an orthopaedic sole wherein the protective textile material is a polyamide non-stretch non-woven fabric and the silicone elastomer is of the RTV-2 type having a Shore hardness A of less than 50.

14. An orthopaedic device according to claim 12, consisting of a knee joint orthosis wherein the textile material is a woven or knitted stretch fabric and wherein the silicone elastomer is an RTV-2-type elastomer having a Shore hardness A of less than 50.
